# EUROPEAN PATENT APPLICATION

(11) **EP 1 728 789 A1**
(43) Date of publication of application: **06.12.2006**
(21) Application number: 04723048.7
(22) Date of filing: 24.03.2004
(51) Int. Cl.: C07D 309/38, C12P 17/06, A61K 31/366, A61P 33/00, A61P 35/00

(54) **ANTIBIOTIC FKI-1778 AND PROCESS FOR PRODUCING THE SAME**

(71) Applicant: THE KITASATO INSTITUTE, Tokyo 108-8642 (JP)
(72) Inventor: OMURA, Satoshi, Setagaya-ku, Tokyo 157-0076 (JP); TOMODA, Hiroshi, Chofu-shi, Tokyo 182-0034 (JP); MASUMA, Rokuro, Minato-ku, Tokyo 108-0073 (JP)
(74) Representative: Duckworth, Timothy John
(86) International application number: PCT/JP2004/004137
(87) International publication number: WO 2005/090326

(57) **Abstract**

The present invention is comprised of culturing a microorganism belonging to fungi and having ability to produce antibiotic FKI-1778A and/or antibiotic FKI-1778B and/or antibiotic FKI-1778C and/or antibiotic FKI-1778D, accumulating the antibiotic FKI-1778A and/or antibiotic FKI-1778B and/or antibiotic FKI-1778C and/or antibiotic FKI-1778D in the cultured mass and isolating the antibiotic FKI-1778A and/or antibiotic FKI-1778B and/or antibiotic FKI-1778C and/or antibiotic FKI-1778D from said cultured mass. The thus obtained substance is expected as pharmaceuticals, animal drugs and agricultural chemicals, or as pharmaceuticals having growth inhibitory activities against cancer cells.

## Description

### Technical Field

The present invention relates to novel antibiotic FKI-1778 useful for pharmaceuticals having growth inhibitory activity against, for example, nematodes and arthropod, animal drugs, agricultural chemicals, and pharmaceuticals exhibiting growth inhibitory activity against cancer cells, and a process for production thereof.

In the present invention, antibiotic FKI-1778 means a generic name including FKI-1778A substance, FKI-1778B substance, FKI-1778C substance and FKI-1778D substance.

### Background art

Heretofore, large numbers of antibiotics produced by microorganisms have been found, and some of the antibiotics, such as penicillin, streptomycin, amphotericin B, tylosin, monensin, ivermectin, blasticidin S and polyoxin, were used practically in the fields of pharmaceuticals, animal drugs and agricultural chemicals, or anticancer agents such as mitomycin C, bleomycin, doxorubicin, aclarubicin and adriamycin were used practically in the fields of pharmaceuticals (UENO, Yoshio and OHMURA, Satoshi, Ed. "Microbial Medicinal Chemistry", Revised 3rd. Ed., page 179-227, Nankodo Pub., 1995).

There may be some problems on these antibiotics such as toxicities, side effects and resistances, hence developments of novel antibiotics which dissolve these problems are strongly needed.

### Disclosure of the invention

We have studied and continued exploration of antibiotics from the culture mass of microorganisms in order to obtain antibiotics which can solve various problems on the points of toxicity, side effect and resistance, and found that antibiotic FKI-1778 represented by the following formula [I]: 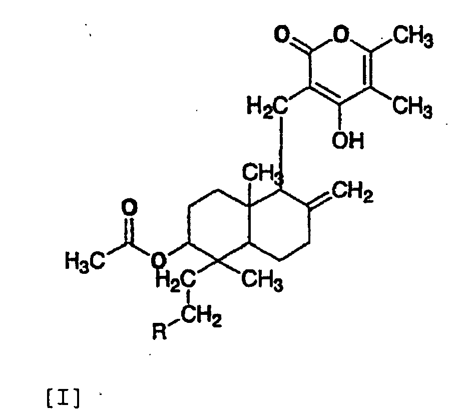 wherein R is 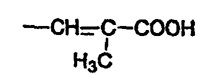 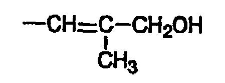 or 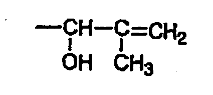 produced by a strain of fungus FKI-1778 had growth inhibitory activities against arthropod and terminating activity on G1 phase of cell cycle against cancer cells, and based on such knowledge, we have completed the present invention.

An object of the present invention is to provide novel antibiotic FKI-1778, which can solve various problems on toxicity, side effect and resistance, and a process for production thereof.

We have continued extensively studies on metabolites produced by microorganisms, and found that substances having growth inhibitory activities were produced in a cultured mass of the newly isolated microorganism, FKI-1778 strain, from soil. Subsequently, as a result of isolating and purifying active substances from the cultured mass, we have found the substances having chemical structure represented by the formula [II], [III], [IV] and [V] hereinafter mentioned. Since such substances have never been known before, these are designated as antibiotic FKI-1778A, antibiotic FKI-1778B, antibiotic FKI-1778C, and antibiotic FKI-1778D.

The present invention has been completed according to such knowledge, and provides antibiotic FKI-1778A represented by the following formula [II]:

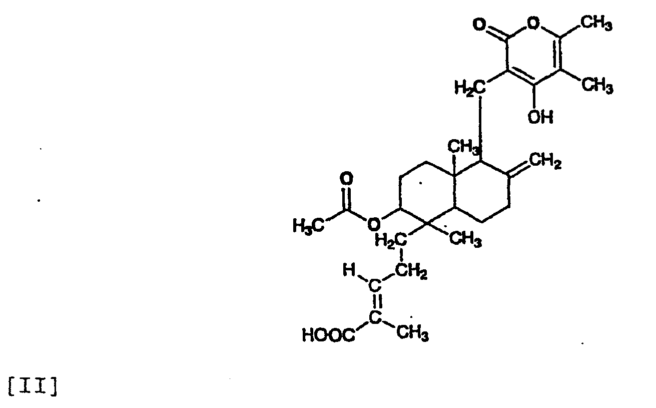

The present invention also provides antibiotic FKI-1778B represented by the following formula [III]:

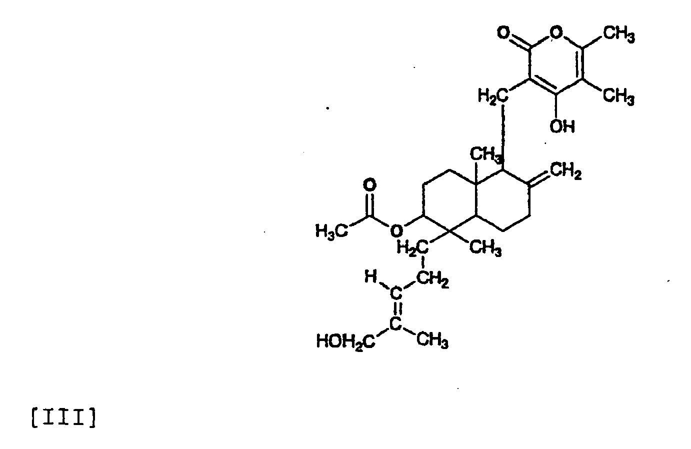

The present invention further provides antibiotic FKI-1778C represented by the following formula [IV]:

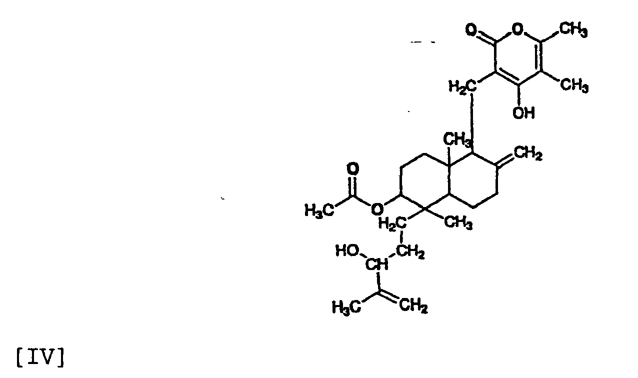

The present invention further provides antibiotic FKI-1778D represented by the following formula [V]:

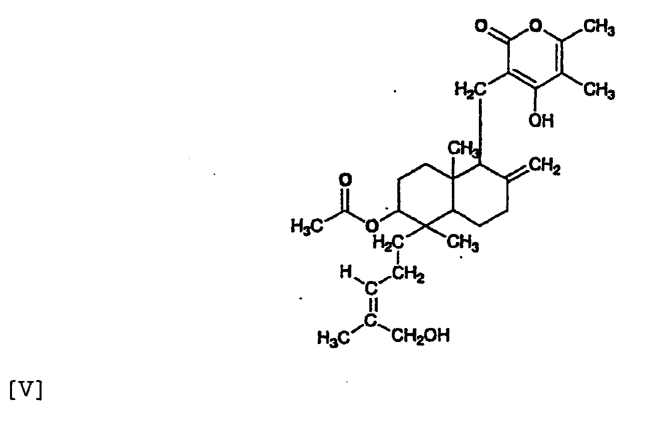

The present invention further provides a process for production of antibiotic FKI-1778 comprising culturing a microorganism belonging to fungus and having ability to produce antibiotic FKI-1778 in a medium, accumulating the antibiotic FKI-1778 in the cultured mass, and isolating FKI-1778 from said cultured mass.

The present invention further provides a process for production of antibiotic FKI-1778 comprising culturing a microorganism belonging to fungus and having ability to produce antibiotic FKI-1778A and/or antibiotic FKI-1778B and/or antibiotic FKI-1778C and/or antibiotic FKI-1778D in a medium, accumulating the antibiotic FKI-1778A and/or antibiotic FKI-1778B and/or antibiotic FKI-1778C and/or antibiotic FKI-1778D in the cultured mass, and isolating the antibiotic FKI-1778A and/or antibiotic FKI-1778B and/or antibiotic FKI-1778C and/or antibiotic FKI-1778D from said cultured mass.

Further, the present invention provides a microorganism belonging to fungus and having ability to produce antibiotic FKI-1778, wherein the microorganism is Albophoma sp. FKI-1778 FERM BP-08668.

The microorganism having ability to produce antibiotic FKI-1778A, antibiotic FKI-1778B, antibiotic FKI-1778C and antibiotic FKI-1778D represented by the formula [II], [III], [IV] and [V] hereinbefore (hereinafter designates as"FKI-1778 substance producing microorganism") belongs to fungus, and can be a microorganism having ability to produce the substance of the present invention, and is not limited. Preferable example of the microorganism strain used for production of the antibiotic FKI-1778 of the present invention is a strain of fungus FKI-1778 which was newly isolated from a soil sample in Amami-Oshima, Kagoshima Pref., Japan by the present inventors.

Taxonomical properties of the strain of Albophoma sp. FKI-1778 of the present invention are as follows.

### 1. Morphological properties:

The present strain shows good growth in potato glucose agar medium, malt extract agar medium, corn meal agar medium and Miura agar medium, and also shows good growth of conidiospore in potato glucose agar medium, corn meal agar medium and Miura agar medium. In the malt extract agar medium, formation of conidiospore is slightly suppressive.

Color of conidiocarp is white and occasionally covered with aerial mycelia. Conidiocarp consists of thin net-like structured tissues with intricate hyphae and no ostioles are observed. The form is globose to subglobose, and grows on the medium with the size of 100 - 300 µm.

In the conidiocarp, many conidia are observed with the form of colorless globose in the size of 1.6 - 2.2 µm.

### 2. Culture properties on various agar medium

Results of macroscopic observation cultured on potato glucose agar medium, malt extract agar medium, corn meal agar medium and Miura agar medium at 25°C for 2 weeks are shown in Table 1.

**Table 1**

| Medium | Growth condition on medium (diameter of colony) | Color of surface of colony | Color of reverse of colony | Soluble pigment |
|---|---|---|---|---|
| Potato - glucose agar | Good (72-74 mm), floccose, corrugate, entire | White | Pale cream | None |
| Malt extract agar | Good (72-73 mm), floccose, centrally raised, entire | White - pale cream | Cream - ivory | None |
| Corn meal agar | Good (70-71 mm), Thin floccose, entire | White - pale cream | White - pale cream | None |
| Miura's agar | Good (50-60 mm), Thin floccose, Irregular | White | White | None |

### 3. Physiological properties

(1) Optimum growth condition
   Optimum growth condition of the strain is pH 4.0 - 7.0 and temperature at 15.0 - 28.5°C.
(2) Growth range
   Growth range of the strain is pH 3.0 - 8.0 and temperature at 7.5 - 34.0°C.
(3) Aerobic or anaerobic nature
   Aerobic.

Based on the above taxonomical properties, culture properties and physiological properties of the strain FKI-1778, these properties were compared with known microorganism strains, and the present strain was referred to the strain belonging to genus Albophoma sp. and was designated as Albophoma sp. FKI-1778. This strain was deposited with the name of Albophoma sp. FKI-1778 in the International Patent Organism Depository, National Institute of Advanced Industrial Science and Technology, AIST Tsukuba Central 6, 1-1, Higashi 1-Chome, Tsukuba-shi, Ibaraki-ken, 305-8566 Japan and given a permanent depository number as FERM BP-08668 on March 22, 2004.

Although the strain of Albophoma sp. FKI-1778 can be mentioned as the preferable producing microorganism strain in the antibiotic FKI-1778 producing microorganisms of the present invention, it is well known that microorganisms are very easy to mutate and the taxonomical properties are not constantly maintained as the general properties of microorganisms, further microorganisms are mutated by means of natural or common artificial mutation, for example ultraviolet irradiation or use of mutant inducer such as N-methyl-N'-nitro-N-nitrosoguanidine, ethyl methansulfonate, etc. Consequently, all microorganism strains including such artificial mutant strains and natural mutant strains belonging to fungi and having producing ability of antibiotic FKI-1778 represented by the formula [I] hereinbefore can be used in the present invention.

In the production of antibiotic FKI-1778 of the present invention, the antibiotic FKI-1778 producing strain belonging to fungi is cultured in the medium and the antibiotic FKI-1778 is isolated and purified. Examples of nutrient sources preferable for production of the antibiotic FKI-1778 can be materials which can be used for nutrient sources of fungi. For example, nitrogen sources such as commercially available peptone, meat extract, corn steep liquor, cotton seed oil, peanuts powder, soybean powder, yeast extract, NZ-amine, casein hydrolyzate, sodium nitrate, ammonium nitrate and ammonium sulfate, carbohydrates such as glycerol, starch, glucose, galactose and mannose, carbon sources such as fat, and inorganic salts such as sodium chloride, phosphate, calcium carbonate and magnesium sulfate can be used alone or in combination.

If necessary, trace amounts of metal salt, and antifoam agents such as animal, vegetable or silicone oil can be added. These substances can be substances useful for production of antibiotic FKI-1778 by the producing microorganism, and all known materials for culturing fungi can be used. For mass production of antibiotic FKI-1778, liquid culture is preferable, and the culturing temperature, which can be applied, is ranges within growth of the producing microorganism and production of antibiotic FKI-1778. The culturing conditions can be advantageously selected depending on the properties of antibiotic FKI-1778 producing microorganisms using the above described conditions.

Antibiotic FKI-1778 can be extracted by water immiscible organic solvents such as chloroform and ethyl acetate from the cultured liquid. In addition to the above extraction method, known methods for isolation of lipophilic substances, for example, adsorption chromatography, gel filtration chromatography, scrape out from thin layer chromatography, centrifugal counter-current chromatography and high performance liquid chromatography, can be used in combination or repeatedly to isolate the purified product.

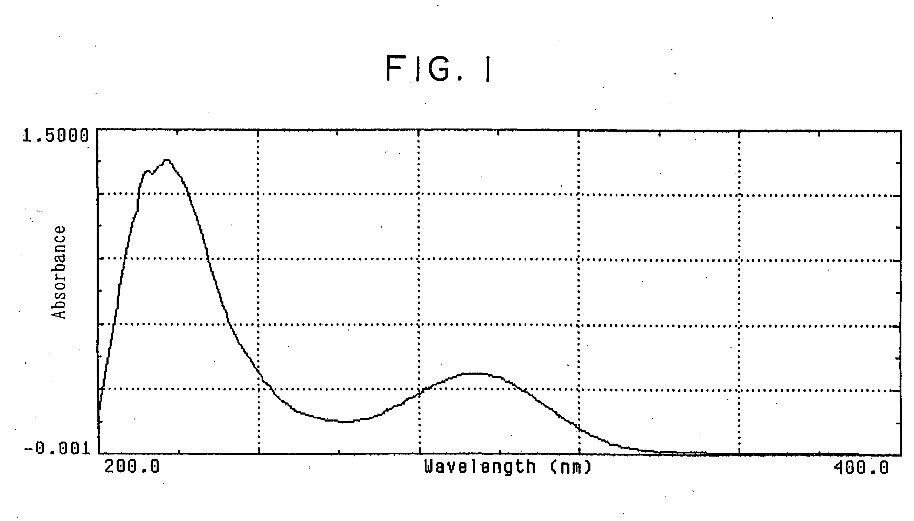

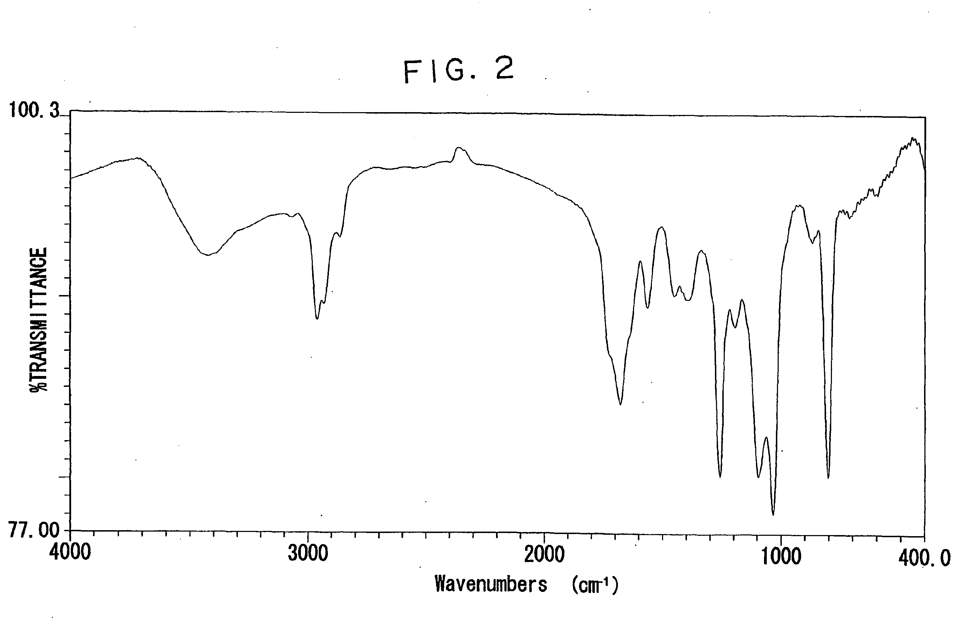

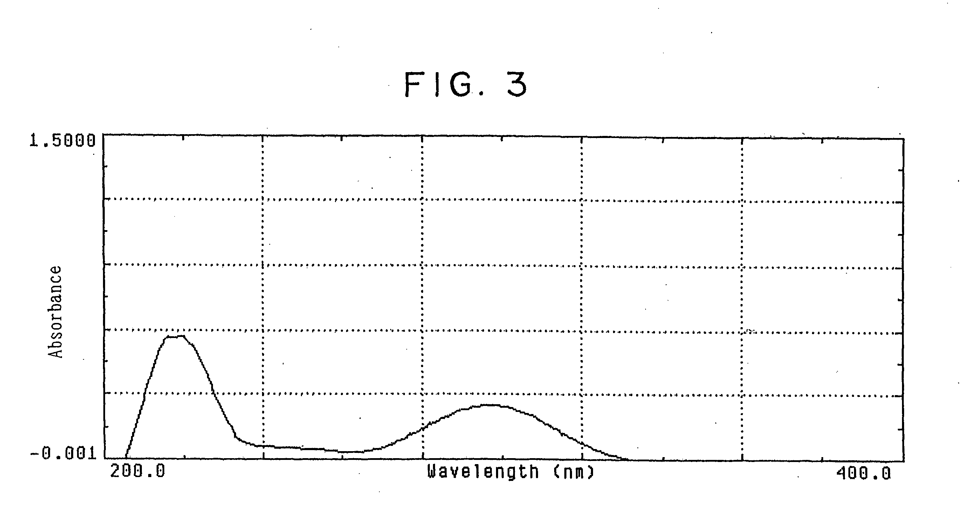

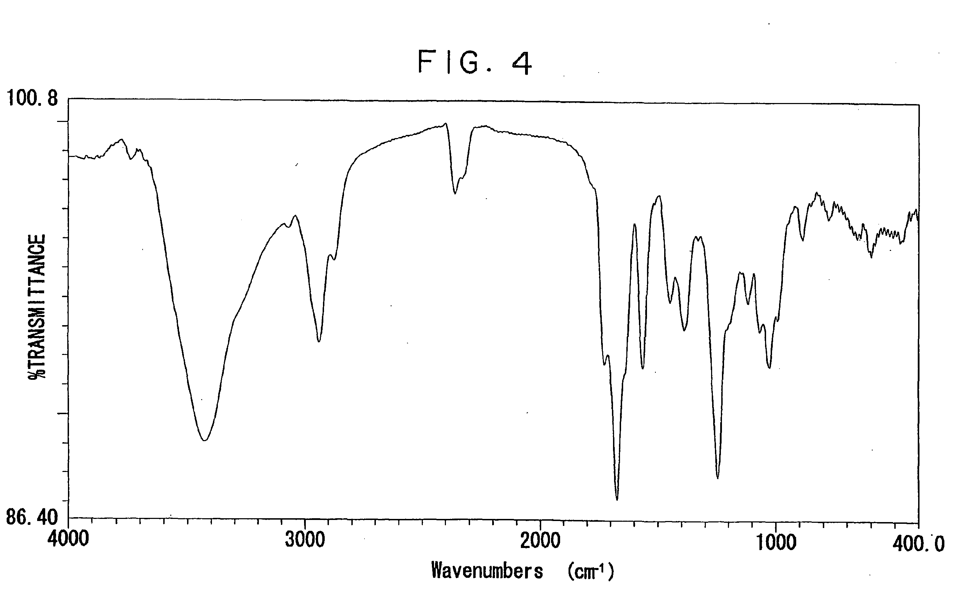

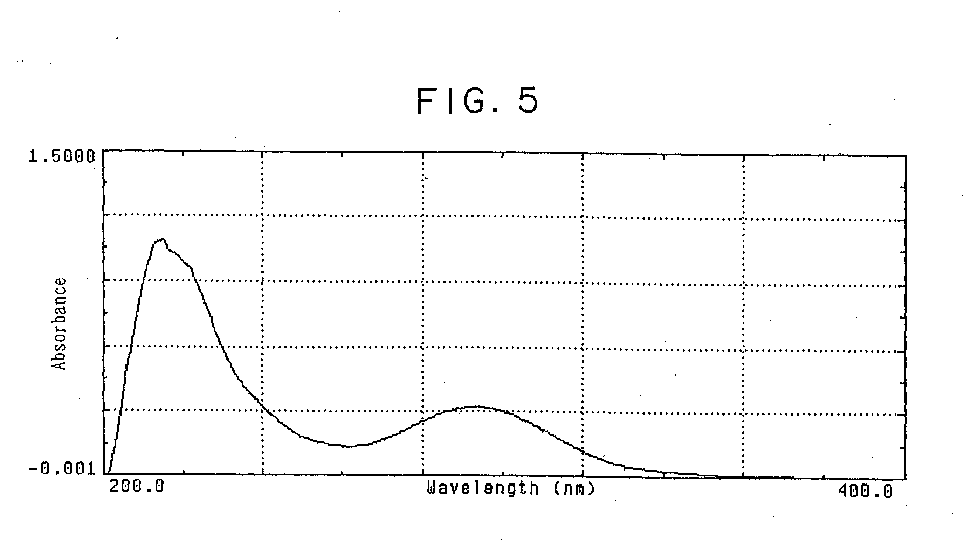

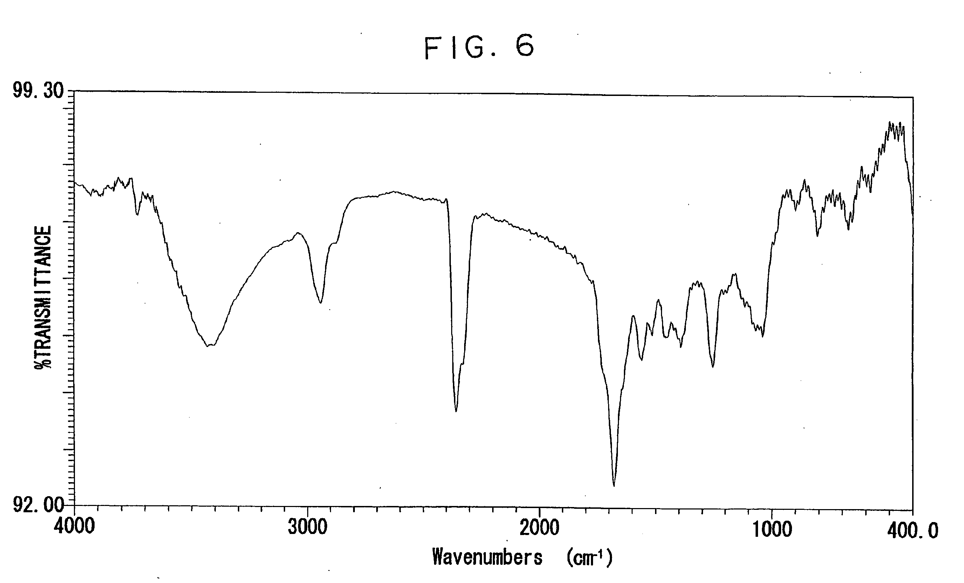

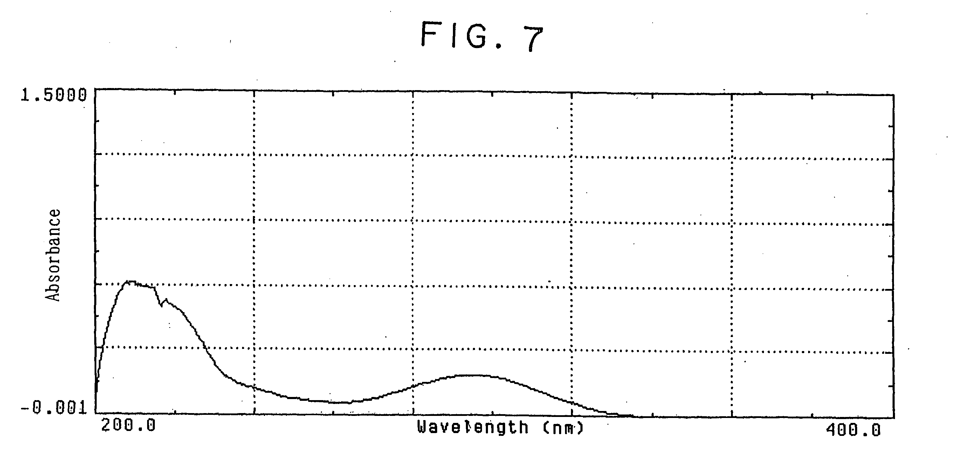

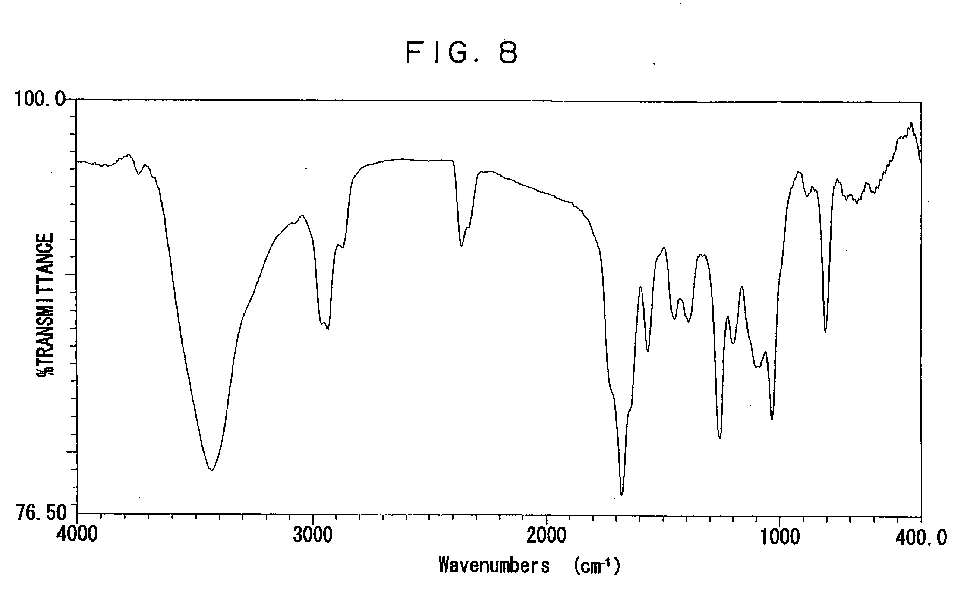

Physicochemical properties of antibiotic FKI-1778A, antibiotic FKI-1778B, antibiotic FKI-1778C and antibiotic FKI-1778D are as follows.
[I] Antibiotic FKI-1778A
   (1) Nature: pale yellow powder
   (2) Molecular weight: 501 (M+H, fast atom bombardment mass spectrometry)
   (3) Molecular formula: C₂₉H₄₀O₇
   (4) Ultraviolet absorption spectrum: UV spectrum measured in methanol is as shown in Fig. 1, and has specific absorption peaks λmax at 294 nm.
   (5) Infrared absorption spectrum: IR spectrum measured by KBr tablet is as shown in Fig. 2, and has specific absorption peaks λmax at 3430, 2960, 1687, 1564, 1450, 1390, 1259, 1097, 1036 and 806 cm⁻¹.
   (6) Proton nuclear magnetic resonance spectrum: Chemical shifts in deuterated methanol (ppm) and spin coupling constants (Hz) are shown as follows.
      1.89m(1H), 1.35m(1H), 1.84m(1H), 1.73m(1H), 4.87m(1H), 1.84br.d(1H, J=15.2), 1.56m(1H), 1.35m(1H), 2.47dt(1H, J=6.0, 14.0), 2.10br.d(1H J=14.0), 2.20m(1H), 2.78t(1H, J=12.6), 2.61dd(1H, J=4.2, 12.6), 4.52m(1H), 4.25m(1H), 1.02s(3H), 0.93s(3H), 1.42m(1H), 1.26m(1H), 2.22m(1H), 2.18m(1H), 6.73t(1H, J=7.3), 1.82s(3H), 1.93s(3H), 2.21s(3H), 2.05s(3H)
      s: singlet, d: doublet, t: triplet, q: quadplet, m: multiplet, br: broad, H: number of proton, J: spin coupling constant (Hz).
   (7) ¹³C nuclear magnetic resonance spectrum: Chemical shifts in deuterated methanol (ppm) are shown as follows.
      35.0t, 25.1t, 77.3t, 41.3s, 40.4d, 23.8t, 32.1t, 149.7s, 56.2d, 38.6s, 22.6t, 110.6t, 23.6q, 18.6q, 37.2t, 23.5t, 143.7d, 128.7s, 171.6s, 12.3q, 168.1s, 104.0s, 167.7s, 108.7s, 156.9s, 10.4q, 17.2q, 172.6s, 21.1q
   (8) Solubility in solvents: Soluble in chloroform, ethyl acetate, acetone, methanol and acetonitrile. Slightly soluble in water and h-hexane.
   (9) Color reaction: Brownish colored in sulfuric acid.
[II] Antibiotic FKI-1778B
   (1) Nature: pale yellow powder
   (2) Molecular weight: 487 (M+H, fast atom bombardment mass spectrometry)
   (3) Molecular formula: C₂₉H₄₂O₆
   (4) Ultraviolet absorption spectrum: UV spectrum measured in methanol is as shown in Fig. 3, and has specific absorption peaks λmax at 297 nm.
   (5) Infrared absorption spectrum: IR spectrum measured by KBr tablet is as shown in Fig. 4, and has specific absorption peaks λmax at 3430, 2938, 2360, 1725, 1673, 1563, 1448, 1388, 1247, 1146, 1028 and 887 cm⁻¹.
   (6) Proton nuclear magnetic resonance spectrum: Chemical shifts in deuterated methanol (ppm) and spin coupling constants (Hz) are shown as follows.
      1.85m(1H), 1.34m(1H), 1.37m(1H), 1.71m(1H), 4.87dd(1H, J=4.5, 11.4), 1.85dd(1H, J=2.4, 15.6), 1.56m(1H), 1.36m(1H), 2.46dt(1H, J=5.4, 13.8), 2.06br.d(1H, J=13.8), 2.20m(1H), 2.77t(1H, J=12.6), 2.80dd(1H, J=4.8, 12.6), 4.51m(1H), 4.23m(1H), 1.01s(3H), 0.91s(3H), 1.38m(1H), 1.22m(1H), 2.09m(1H), 2.07m(1H), 5.36t(1H, J=7.2), 3.91s(2H), 1.56s(3H), 1.93s(3H), 2.21s(3H), 2.03q(3H)
   (7) ¹³C nuclear magnetic resonance spectrum: Chemical shifts in deuterated methanol (ppm) are shown as follows.
      35.0t, 25.1t, 77.5t, 41.2s, 40.4d, 23.8t, 32.1t, 149.9s, 56.2d, 38.6s, 22.4t, 110.6t, 23.6q, 18.7q, 38.5t, 22.5t, 126.8d, 135.9s, 69.0t, 13.6q, 168.1s, 104.0s, 167.7s, 108.7s, 156.9s, 10.4q, 17.2q, 172.6s, 21.1q
   (8) Solubility in solvents: Soluble in chloroform, ethyl acetate, acetone, methanol and acetonitrile. Slightly soluble in water and h-hexane.
   (9) Color reaction: Brownish colored in sulfuric acid.
[III] Antibiotic FKI-1778C
   (1) Nature: pale yellow powder
   (2) Molecular weight: 485 (M+H, fast atom bombardment mass spectrometry)
   (3) Molecular formula: C₂₉H₄₂O₆
   (4) Ultraviolet absorption spectrum: UV spectrum measured in methanol is as shown in Fig. 5, and has specific absorption peaks λmax at 293 nm.
   (5) Infrared absorption spectrum: IR spectrum measured by KBr tablet is as shown in Fig. 6, and has specific absorption peaks λmax at 3430, 2940, 2327, 1678, 1558, 1461, 1390, 1254, 1081, 1037, and 808 cm⁻¹.
   (6) Proton nuclear magnetic resonance spectrum: Chemical shifts in deuterated methanol (ppm) and spin coupling constants (Hz) are shown as follows.
      1.87br.d(1H, J=13.2), 1.34m(1H), 1.82m(1H), 1.72m(1H), 4.76m(1H), 1.77br.d(1H, J=12.6), 1.57m(1H), 1.37m(1H), 2.44dt(1H, J=5.8, 14.0), 2.08br.d(1H, J=13.2), 2.17dd(1H, J=3.6, 12.6), 2.77t(1H, J=12.0), 2.59dd(1H, J=3.6, 12.0), 4.51m(1H), 4.23m(1H), 1.00s(3H), 0.91s(3H), 1.37m(1H), 1.13m(1H), 1.55m(1H), 1.49m(1H), 3.87t(1H, J=6.0), 1.69s(3H), 4.89m(1H), 4.83m(1H), 1.93s(3H), 2.20s(3H), 2.02s(3H)
   (7) ¹³C nuclear magnetic resonance spectrum: Chemical shifts in deuterated methanol (ppm) are shown as follows.
      35.0t, 25.1t, 77.7t, 41.5t, 40.6d, 23.8t, 32.1t, 150.0s, 56.2d, 38.6s, 22.5t, 110.6t, 23.7q, 18.8q, 35.1t, 29.3t, 77.8d, 148.8s, 17.5q, 111.9s, 168.4s, 104.1s, 167.9s, 108.7s, 157.0s, 10.4q, 17.2q, 172.7s, 21.1q
   (8) Solubility in solvents: Soluble in chloroform, ethyl acetate, acetone, methanol and acetonitrile. Slightly soluble in water and h-hexane.
   (9) Color reaction: Brownish colored in sulfuric acid.
[IV] Antibiotic FKI-1778D
   (1) Nature: pale yellow powder
   (2) Molecular weight: 487 (M+H, fast atom bombardment mass spectrometry)
   (3) Molecular formula: C₂₉H₄₂O₆
   (4) Ultraviolet absorption spectrum: UV spectrum measured in methanol is as shown in Fig. 7, and has specific absorption peaks λmax at 295 nm.
   (5) Infrared absorption spectrum: IR spectrum measured by KBr tablet is as shown in Fig. 8, and has specific absorption peaks λmax at 3430, 2933, 2358, 1677, 1563, 1450, 1388, 1257, 1197, 1085, 1033 and 806 cm⁻¹.
   (6) Proton nuclear magnetic resonance spectrum: Chemical shifts in deuterated methanol (ppm) and spin coupling constants (Hz) are shown as follows.
      1.82m(1H), 1.34m(1H), 1.87m(1H), 1.71m(1H), 4.86dd(1H, J=4.2, 11.4), 1.82m(1H), 1.56m(1H), 1.36m(1H), 2.46dt(1H, J=4.8, 13.2), 2.09br.d(1H, J=13.2), 2.19m(1H), 2.77dd(1H, J=12.0, 12.6), 2.59dd(1H, J=4.2, 12.6), 4.51m(1H), 4.23m(1H), 1.01s(3H), 0.91s(3H), 1.33m(1H), 1.19m(1H), 2.10m(1H), 2.08m(1H), 5.23t(1H, J=7.2), 4.07s(2H, J=12.0, 16.2), 1.76s(3H), 1.93s(3H), 2.21s(3H), 2.03q(3H)
   (7) ¹³C nuclear magnetic resonance spectrum: Chemical shifts in deuterated methanol (ppm) are shown as follows.
      35.0t, 25.1t, 77.5t, 41.2t, 40.4d, 23.8t, 32.1t, 149.9s, 56.2d, 38.6s, 22.4t, 110.6t, 23.6q, 18.7q, 38.5t, 21.8t, 127.5d, 134.4s, 60.0t, 20.3q, 168.1s, 104.0s, 167.7s, 108.7s, 156.9s, 10.4q, 17.2q, 172.6s, 21.1q
   (8) Solubility in solvents: Soluble in chloroform, ethyl acetate, acetone, methanol and acetonitrile. Slightly soluble in water and h-hexane.
   (9) Color reaction: Brownish colored in sulfuric acid.

Next, diameters of inhibition ring of antibiotic FKI-1778A, antibiotic FKI-1778B, antibiotic FKI-1778C and antibiotic FKI-1778D against various microorganisms on the nutrient agar medium are shown in Table 2.

**Table 2**

| Test organisms | Diameter of inhibition ring (mm) | | | |
|---|---|---|---|---|
| | A | B | C | D |
| Staphylococcus aureus ATCC6538p | - | - | - | - |
| Bacillus subtilis ATCC6633 | - | - | - | - |
| Micrococcus luteus ATCC9341 | - | - | - | - |
| Mycobacterium smegmatis ATCC607 | - | - | - | - |
| Escherichia coli NIHJ | - | - | - | - |
| Escherichia coli NIHJ JC-2(IFO12734) | - | - | - | - |
| Pseudomonas aeruginosa IFO3080 | - | - | - | - |
| Xanthomonas campestris pv. oryzae KB88 | - | - | - | - |
| Candida albicans KF1 | - | - | - | - |
| Saccharomyces cerevisiae KF26 | - | - | - | - |
| Aspergillus niger ATCC6275 | - | - | - | - |
| Mucor racemosus IF04581 | - | - | - | - |

As obvious from Table 2, antibiotic FKI-1778A, antibiotic FKI-1778B, antibiotic FKI-1778C and antibiotic FKI-1778D of the present invention exhibit no growth inhibitory activities against various microorganisms.

Next, anti-arthropod activities of antibiotic FKI-1778 of the present invention are described hereinbelow.

Methanol solution of each antibiotic FKI-1778 was placed in the microplate (96 wells) (Corning Corp., the U.S.). After distilled off methanol in vacuo, the test medium 250 µl (lecithin 0.01%, sodium hydrogen carbonate 7.5 mM, potassium chloride 7.5 mM, calcium chloride dihydrate 7.5 mM and magnesium sulfate heptahydrate 7.5 mM) was added and stirred for 15 minutes.

The buffer solution 50 µl containing several numbers of nauplius larva of Artemia salina, Arthropod, which was incubated in the buffer solution (Tris 20 mM, sodium chloride 440 mM, magnesium chloride 23 mM, sodium carbonate 1.9 mM, magnesium sulfate 53 mM and calcium chloride 10 mM), was added to each well, and the condition thereof was observed microscopically after 2 days. Results are shown in Table 3.

**Table 3**

| Antibiotic | Anti-arthropod activity (µg/ml) |
|---|---|
| FKI-1778A | >200 |
| FKI-1778B | 100 |
| FKI-1778C | >200 |
| FKI-1778D | 100 |

As obvious from the Table 3, antibiotic FKI-1778 of the present invention exhibits growth inhibitory activities against Arthropod and can be used as medicaments such as larvicide. The arthropods have a segmented body with appendages on each segment. All arthropods are covered by a hard exoskeleton that is made out of chitin, a polysaccharide, and include centipede, mite, spider, crab and shrimp.

Further, a terminating activity of antibiotic FKI-1778 of the present invention against cell cycle G1 phase in cancer cells is explained as follows.

Using 96-well microplate (Corning Corp., the U.S.), human leukemia cell strain Jurkat cell, 5 × 10⁵ cells/well, was inoculated into RPMI 1640 medium (IWAKI Corp., Japan) 200 µl containing a mixture of 10% fetal bovine serum and 1% penicillin (10,000 units/ml) - streptomycin (10 mg/ml) (Invitrogen Inc., the U.S.) and cultured. Antibiotic FKI-1778, 5 µg, was added thereto, and the mixture was incubated at 37°C for 24 hours under 5% CO₂ atmosphere, and the culture supernatant was discarded. Cells were suspended in 0.1% aqueous sodium citrate solution (200 µl) containing 0.002% ribonuclease A (Sigma Corp., the U.S.), 0.005% propidium iodide (Sigma Corp., the U.S.) and 0.3 ml/100 ml IGEPAL-CA-630 (Sigma Corp., the U.S.) . After the cell suspension was allowed to stand for 2 hours or more, DNA contents in 10,000 cells were measured by using flow cytometer (FACS Calibur, Becton, Dickinson and Company, the U.S.), then DNA contents of cells in G1 phase, S phase and G2/M phase were measured by using software, Cell Quest (Becton, Dickinson and Company, the U.S.). Results are shown in Table 4.

**Table 4**

| Antibiotics | G1 phase (%) | G2/M phase (%) |
|---|---|---|
| FKI-1778A | 62.4 | 12.8 |
| FKI-1778B | 55.0 | 12.0 |
| FKI-1778C | 64.2 | 13.0 |
| FKI-1778D | 63.9 | 9.9 |
| No addition | 53.2 | 22.3 |

As demonstrated in Table 4, since antibiotic FKI-1778 exhibits cell cycle G1 phase terminating activity against human leukemia cell strain Jurkat cell, it can be used as anticancer agent.

### Brief description of drawings

Fig. 1 shows ultraviolet absorption spectrum of antibiotic FKI-1778A of the present invention.
Fig. 2 shows infrared absorption spectrum of antibiotic FKI-1778A of the present invention.
Fig. 3 shows ultraviolet absorption spectrum of antibiotic FKI-1778B of the present invention.
Fig. 4 shows infrared absorption spectrum of antibiotic FKI-1778B of the present invention.
Fig. 5 shows ultraviolet absorption spectrum of antibiotic FKI-1778C of the present invention.
Fig. 6 shows infrared absorption spectrum of antibiotic FKI-1778C of the present invention.
Fig. 7 shows ultraviolet absorption spectrum of antibiotic FKI-1778D of the present invention.
Fig. 8 shows infrared absorption spectrum of antibiotic FKI-1778D of the present invention.

### Best mode for carrying out the invention

The present invention is explained by mentioning example, but is not construed as limiting within the example.

### Example

One loopful strain of Albophoma sp. FKI-1778 FERM BP-08668 cultured on the agar slant was inoculated into the liquid medium (pH 6.0) 100 ml consisting of glucose 2.0%, polypeptone (Nihon Seiyaku Co., Japan) 0.5%, yeast extract (Oriental Yeast Co., Japan) 0.2%, agar 0.1%, potassium dihydrogenphosphate 0.1% and magnesium sulfate heptahydrate 0.05% in a 500 ml Erlenmeyer flask, and shake cultured at 27°C for 3 days. The cultured seed culture liquid 1 ml was inoculated into each one of 500 ml Erlenmeyer flasks, total 60 flasks, containing a liquid medium, each 100 ml, consisting of glycerol 3.0%, oatmeal 2.0%, dry yeast 1.0%, potassium hydrogenphosphate 1.0%, disodium hydrogenphosphate 1.0% and magnesium sulfate heptahydrate 0.05%, and shake cultured at 27°C for 6 days.

The cultured mass was separated to a supernatant and mycelia by centrifugation, and the supernatant was extracted with ethyl acetate and concentrated in vacuo to obtain crude substance I 814 mg. The substance was dissolved in methanol and the soluble fraction was used as the fixed bed of the upper layer and the moving bed of the lower layer in the two layered system of hexane - methanol (2 : 1). The solution was charged on the liquid-liquid partition chromatography (Miki K.K., Japan) and eluted by normal elution. The fraction was concentrated in vacuo to obtain crude substance of FKI-1778, 98.3 mg. FKI-1778 was subjected to HPLC (Xtera ODS, φ19 × 100 mm, Waters Inc., the U.S.) with a mobile phase of 40% acetonitrile and peaks were collected, and the collected fractions were concentrated in vacuo to isolate FKI-1778A, 4.4 mg, FKI-1778B, 18.6 mg, FKI-1778C, 2.5 mg, and FKI-1778D, 3.6 mg.

### Industrial applicability

As explained hereinabove, the microorganism belonging to fungi and having ability to produce antibiotic FKI-1778 is cultured in a medium, and antibiotic FKI-1778 is accumulated in the medium, then antibiotic FKI-1778 is collected from the cultured mass. The thus obtained antibiotic FKI-1778 is novel antibiotic useful as insecticide or anticancer agent. The present antibiotic is produced from fungi FKI-1778 strain, and is expected to be useful as pharmaceuticals, animal drugs and agricultural chemicals.

## Claims

1. Antibiotic FKI-1778 represented by the following formula [I] : 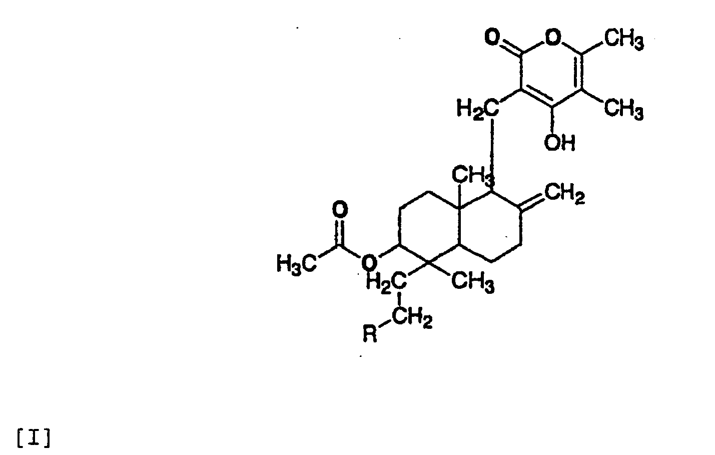 wherein R is 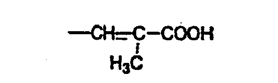 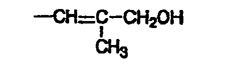 or

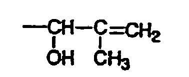

2. A composition FKI-1778 comprising FKI-1778A represented by the following formula [II]: 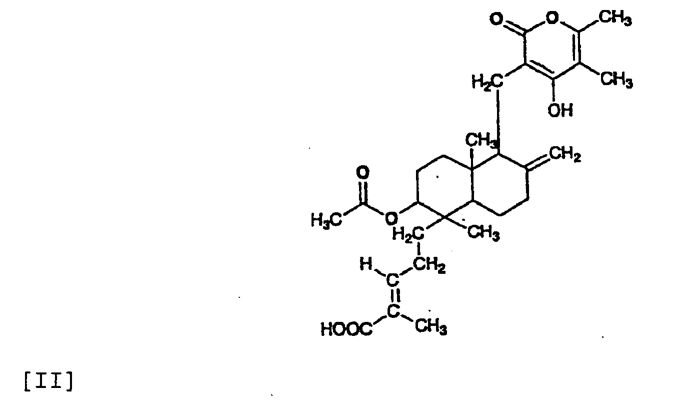 and/or FKI-1778B represented by the following formula [III]: 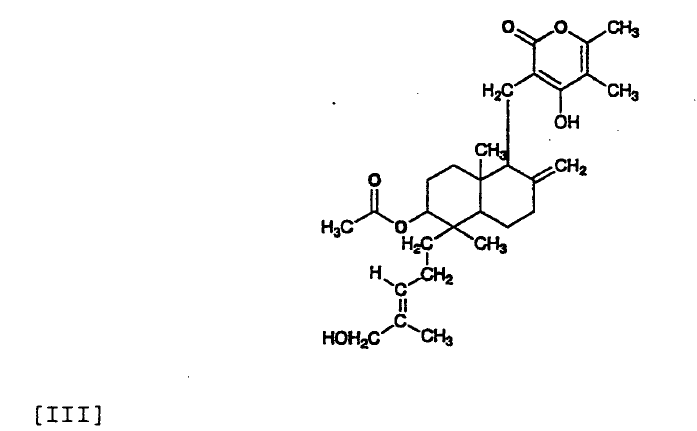 and/or FKI-1778C represented by the following formula [IV]: 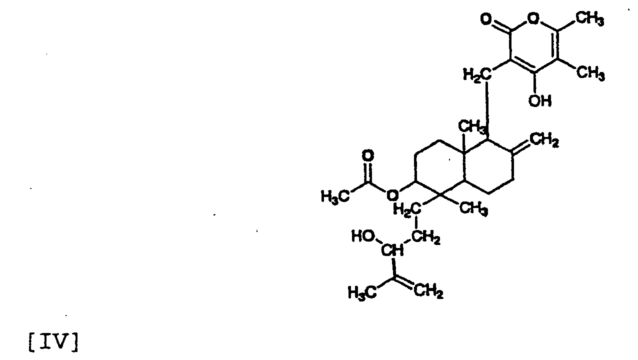 and/or FKI-1778D represented by the following formula [V]:

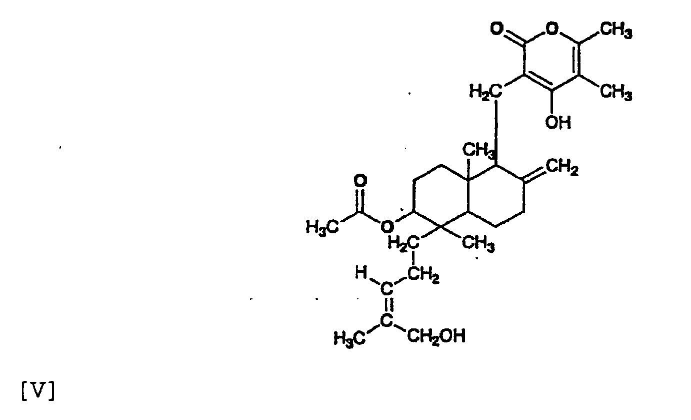

3. A process for production of antibiotic FKI-1778 comprising culturing a microorganism belonging to fungi and having ability to produce antibiotic FKI-1778, accumulating the antibiotic FKI-1778 in the cultured mass and isolating the antibiotic FKI-1778 from said cultured mass.

4. A process for production of composition of FKI-1778 comprising culturing a microorganism belonging to fungi and having ability to produce antibiotic FKI-1778A and/or antibiotic FKI-1778B and/or antibiotic FKI-1778C and/or antibiotic FKI-1778D, accumulating the antibiotic FKI-1778A and/or antibiotic FKI-1778B and/or antibiotic FKI-1778C and/or antibiotic FKI-1778D in the cultured mass and isolating the antibiotic FKI-1778A and/or antibiotic FKI-1778B and/or antibiotic FKI-1778C and/or antibiotic FKI-1778D from said cultured mass.

5. A microorganism, wherein the microorganism belonging to fungi and having ability to produce antibiotic FKI-1778 is Albophoma sp. FKI-1778 FERM BP-08668, and a mutant thereof.
